# EUROPEAN PATENT SPECIFICATION

(11) **EP 1 811 901 B1**
(45) Date of publication and mention of the grant of the patent: **29.04.2009**
(21) Application number: 04770530.6
(22) Date of filing: 19.09.2004
(51) Int. Cl.: A61B 8/12, G01S 15/89, G01H 9/00

(54) **INTRAVASCULAR ULTRASOUND IMAGING DEVICE**
INTRAVASKULÄRE ULTRASCHALLDARSTELLUNGSVORRICHTUNG
DISPOSITIF D'IMAGERIE INTRAVASCULAIRE A ULTRASONS

(43) Date of publication of application: 01.08.2007
(73) Proprietor: Bioscan, Ltd., 20692 Yokneam Eilit (IL)
(72) Inventor: MATCOVITCH, Avram, 36845 Nesher (IL); KHACHATUROV, Arkady, 32495 Haifa (IL); VOITSECHOV, Yury, 20115 Moshav Amirim (IL); BAR-LEV, Zvi, 37867 M.P. Menashe (IL); JAPHA, Yonathan, 76223 Rehovot (IL); HASSON, Salah, 20200 Shefar'am (IL)
(74) Representative: Machtalère, Georges
(86) International application number: PCT/IL2004/000859
(87) International publication number: WO 2006/030408

(56) References cited:
- WO-A-99/58059
- DE-A1- 3 047 308
- GB-A- 2 176 364
- US-A1- 2002 058 890
- US-A1- 2004 067 000
- US-A1- 2004 131 299
- WIESLER D ET AL: "FIBER OPTIC ULTRASOUND SENSORS FOR MEDICAL IMAGING APPLICATIONS" INTERNATIONAL CONFERENCE ON OPTICAL FIBER SENSORS. ADVANCED SENSING PHOTONICS, 1997, pages 358-361, XP000874814

## Description

### FIELD OF THE INVENTION

The invention relates to apparatus for determining characteristics of a lumen of a conduit, for example of the lumen of a vessel in the body.

### BACKGROUND OF THE INVENTION

To assess an extent to which the lumen of a vessel in a patient's body, such as a blood vessel, a bile duct or the urethra, is damaged by disease and, if damaged, advisability of medical intervention to alleviate and/or correct the damage, it is generally required to assess topology of the lumen. For example, to determine an extent to which atherosclerosis damages a blood vessel in the patient's body, it is generally required to determine blood vessel diameter and where and to what extent the blood vessel is narrowed by atherosclerotic plaque.

Often an intravascular ultrasound (IVUS) catheter is used to provide information characterizing the condition of a region of a blood vessel (or other vessel) in the body and provide topographical information of the blood vessel walls and lumen in the region. Generally, an IVUS catheter comprises at least one ultrasound transducer mounted to a distal end of the catheter that is inserted into the lumen of the blood vessel region to be examined and a controller located at a proximal end of the catheter, which remains outside the body. The controller is coupled to the at least one transducer by suitable power and data lines that lie along the catheter. In order to introduce the distal end of the IVUS catheter into the lumen, usually a guidewire is first inserted into and threaded through the vascular system to the region of the blood vessel to be examined. The IVUS catheter is then threaded over the guidewire until the distal end of the catheter is appropriately located in the region. The controller controls the at least one transducer to radiate ultrasound that is incident on regions of walls of the lumen and structure in and in the neighborhood of the walls. Acoustic energy in the incident ultrasound that is reflected by the walls and structures is received by the at least one transducer, which generates signals responsive to the incident energy and transmits the signals along the data line or lines to the controller. The controller processes the signals it receives to determine characteristics of the lumen and in particular to determine the diameter of the lumen. Processing involves time analyzing the reflected ultrasound to determine arrival times of echoes of the transmitted ultrasound that arrive at the at least one transducer from the lumen walls and structure in the neighborhood of the walls.

Often, the environment in which an IVUS operates is electromagnetically noisy and electromagnetic noise picked up by control and/or data lines in an IVUS catheter reduces signal to noise of the IVUS catheter. The IVUS catheter itself generally contributes to the noise, since to control the at least one transducer comprised in the catheter to radiate ultrasound, relatively high voltage pulses at frequencies of hundreds or thousands of kHz are transmitted to the transducer along the catheter power lines. Space in the catheter required to accommodate power and data lines, as well as a guidewire, also, generally, contributes to limiting how small the catheter radius can be made, which in turn determines a minimum size vessel for which the catheter can be used.

Typically, a conventional IVUS catheter has a diameter equal to or greater than about a one millimeter. Such a relatively large diameter precludes using the IVUS to probe relatively small conduits and blood vessels in the body, such as small blood vessels in the eyes. Also, because of its size, an IVUS catheter cannot in general be used to guide another catheter, such as a balloon therapy catheter or stent implant catheter, through the vascular system to provide real time imaging of a region of a blood vessel in which the other catheter is being used to perform corrective intervention.

PCT Publication WO99/58059 describes "an imaging guidewire" (IG) that "can function as the guidewire of vascular interventions and can enable real time imaging during balloon inflation and stent deployment". The imaging guidewire has a relatively small diameter of less than 1 mm and "preferably less than 0.5 mm". The IG comprises a single mode fiber core comprising at least one fiber Bragg grating (FBG) and a piezoelectric "jacket". Pulsed electrical power is transmitted to the jacket over power lines in the IG to stimulate the jacket to transmit ultrasound waves, which are reflected off the walls of the lumen and generate mechanical deformations in the fiber and thereby in the FBG. Pulsed light at a wavelength reflected by the FBG is transmitted into the fiber at its proximal end and reflected by the FBG so that it returns to the proximal end. The mechanical deformations caused by the reflected ultrasound generate modulations of the reflected light, which are sensed by circuitry coupled to the proximal end of the fiber and used to image the lumen.

US patent application publication 2004/006700, which forms the closest prior art document for the present invention, describes an IG for imaging a lumen that transmits neither electrical power nor electrical signals along its length to image the lumen. The IG comprises an optic fiber having a blazed FBG in its core at or near a distal end of the fiber. The FBG receives light at a first wavelength transmitted along the axis of the fiber core and directs the light so that it exits the core and is incident on an absorber located on the surface of the fiber. The absorber absorbs and converts the incident optical energy to ultrasonic waves that are transmitted into the lumen and reflected by the lumen walls. The fiber also comprises at least one pair of FBGs that operate as an interferometer for light at a second wavelength that is transmitted along the fiber core axis. A first, proximal, FBG of the at least one pair of FBGs partially reflects second wavelength light and a second, distal, FBG of the pair substantially completely reflects second wavelength light. Light at the second wavelength reflected from the proximal and distal FBGs combine and interfere to generate an optical interference signal that is a function of an optical path length between the proximal and distal FBGs. The ultrasound reflected from the lumen walls modulates the optical path length between the proximal and distal FBGs and therefore the interference signal. The interference signal is sensed and processed to determine a radius or diameter of the lumen.

Since a blazed FBG directs light that it receives into a relatively small range of azimuth angles, a given FBG and corresponding absorber illuminate the wall of the lumen with ultrasound in a corresponding relatively narrow range of azimuth angles. In an embodiment of the IG described in the patent the optic fiber is rotated to azimuthally scan the lumen wall and provide a 360° image of a region of the wall. In another embodiment described in the patent, to provide a 360° image of a lumen the IG comprises a plurality of optic fibers bonded to the surface of a solid guidewire. Each optic fiber images a different section of the lumen wall.

### SUMMARY OF THE INVENTION

An aspect of an embodiment of the present invention relates to an improved ultrasound imaging guidewire (IG) that may be used to provide ultrasound images of a lumen and/or function as a guidewire for use in procedures and with devices for which conventional guidewires are used.

An aspect of the invention relates to providing a new method of coupling optical energy propagating along an optical fiber to an ultrasound transducer coupled to the fiber that converts optical energy to ultrasound. In accordance with an embodiment of the invention an IG employs the new method to generate ultrasound for imaging a lumen.

An IG, in accordance with an embodiment of the invention, comprises an optic fiber having a distal end comprising at least one ultrasound transducer. The at least one ultrasound transducer converts optical energy transmitted along the fiber from a proximal end thereof to acoustic energy that it radiates into the lumen of the region. Acoustic energy in the transmitted ultrasound that is reflected by regions of the wall of the lumen generate optical signals in the fiber by modulating light transmitted along the fiber that is reflected at the distal end of the fiber back to the proximal end. The signals are sensed at the proximal end and used to image the lumen. The optic fiber, which transmits optical energy for both generating ultrasonic waves and sensing echoes of the generated ultrasound, is hereinafter referred to as a "dual transmission optic fiber".

In accordance with an embodiment of the invention, the dual transmission fiber comprises an inner, optionally, single mode core and an optically conducting outer, ring core, that surround and is concentric with the inner core. In some embodiments of the invention, the ring core is contiguous with the inner core and acts as cladding for the inner core. In some embodiments of the invention, a cladding layer having an index of refraction that is less than the indices of refraction of the inner and ring core is sandwiched between the inner core and the ring core. The outer ring core is used to transmit optical energy that is converted by the at least one acoustic transducer to ultrasound. The inner core is used to transmit the optical signals responsive to echoes of the transmitted ultrasound reflected by the walls of the lumen.

In accordance with an embodiment of the invention, the at least one acoustic transducer comprises an optically absorbing material adhered to a region of the surface of the ring core that absorbs optical energy transmitted along the ring core and converts the energy into ultrasound. In some embodiments of the invention, the absorber is adhered directly to the surface region of the ring core. In some embodiments of the invention, an optical bandpass filter that transmits light in a particular band of wavelengths of light that is absorbed by the absorber is sandwiched between the absorber and the surface of the ring core. In some embodiments of the invention, a plurality of acoustic transducers are coupled to the ring core. Optionally, an optical filter having a different bandpass is sandwiched between the absorber of each of the plurality of transducers and the ring core.

In accordance with an embodiment of the invention, optical energy is transmitted along the ring core to the absorbing material in pulses of light, hereinafter "power light", that propagate along the ring core in propagation modes characterized by relatively higher order radial indices. (A propagation mode is characterized by radial and angular indices that scale and define the radial and angular dependence respectively of the electromagnetic field that characterizes the propagation mode. The angular index gives the order of the angular symmetry of the field. The radial index is the order of a Bessel function that describes the radial dependence of the field.) The inventors have noted that for light pulses that propagate in higher radial order propagation modes, a relatively large portion of the optical energy in the pulses occupies regions of the ring core close to the outer surface of the core. The increased optical energy density near to the outer surface of the ring core improves absorption of optical energy from the light pulses by the absorber.

In accordance with an embodiment of the invention, higher order propagation modes of power light pulses transmitted into the fiber are excited by controlling a configuration, hereinafter referred to as an "insertion configuration", of power light at the proximal end of the fiber that is used to insert the power light pulses into the fiber. The insertion configuration defines the spatial pattern of optical energy in power light pulses that is incident on the proximal end of the fiber and the distribution of angles of incidence of the incident energy. An optical system that directs power light pulses to the proximal end of the fiber determines the insertion configuration of the power light pulses. Optionally, the optical system configures the insertion configuration so that light in power light pulses is incident on the proximal end of the fiber at angles of incidence that are relatively large. Optionally, the angles of incidence are relatively close to an acceptance angle for the ring core corresponding to the numerical aperture of the core. In some embodiments of the invention, higher order modes of propagation are excited by deforming the fiber. Optionally the fiber is deformed at the proximal end of the fiber. Optionally, the deformation comprises bending the fiber.

In an embodiment of the invention, the optical signals responsive to ultrasound echoes comprise phase changes in light that is transmitted into the inner core at its proximal end and reflected at the distal end of the inner core back to the proximal end. Light inserted into the inner core to sense ultrasound echoes is referred to as "signal light". Changes in the index of refraction of localized regions of the fiber caused by acoustic energy in the echoes incident on the localized regions generate the phase changes, which are detected in the signal light using any of many various sensors and methods known in the art. Optionally, the phase changes are determined using a Sagnac type interferometer.

There is therefore provided in accordance with an embodiment of the present invention, an optic fiber adapted for ultrasound imaging of the lumen of a vessel comprising: an inner core for transmitting light having an index of refraction that changes responsive to acoustic energy incident thereon; a ring core concentric with the inner core for transmitting light; a cladding material between the inner and ring cores that has an index of refraction smaller than the index of refraction of the material from which the inner core is formed; and at least one acoustic transducer comprising absorbing material formed on the surface of the ring core that absorbs optical energy transmitted along the ring core and generates ultrasound responsive thereto.

Optionally, the cladding material has an index of refraction smaller than the index of refraction of the material from which the ring core is formed. Alternatively or additionally, the inner core is a single mode core.

In some embodiments of the invention, the absorber comprises a metal. Optionally, the metal is chosen from the group of metals consisting of: aluminum, copper, silver, gold and titanium. Alternatively or additionally, the absorber comprises a metallic powder dispersed in a binding medium.

In some embodiments of the invention, a transducer of the at least one acoustic transducer comprises a transducer shaped in the form of an annulus concentric with the ring core.

In some embodiments of the invention, the at least one acoustic transducer comprises a plurality of acoustic transducers. Optionally, at least two of the acoustic transducers comprise an optical filter located between the absorbing material and the inner core that transmits light in a wavelength band of light that is absorbed by the absorber and blocks light at wavelengths outside the band of wavelength. Optionally, each of the optical filters of at least two of the plurality of acoustic transducers transmit light in different bands of wavelengths. Optionally, each of the at least two different bands of wavelengths are substantially non-overlapping.

In some embodiments of the invention, the plurality of acoustic transducers are configured in an annular array concentric with the ring core. Optionally, all the acoustic transducers of the plurality of transducers are substantially identical.

In some embodiments of the invention, at least two of the absorbers when excited by a same at least one pulse of light generates ultrasound at different frequencies.

In some embodiments of the invention, the fiber comprises an external sheath concentric with the ring core that provides the fiber with mechanical properties that enables the fiber to be inserted and navigated through a system of connected vessels comprising the vessel to position the at least one acoustic transducer in the lumen.

In some embodiments of the invention, the fiber comprises a first end at which light is inserted into the inner and ring cores. Optionally the fiber comprises an optical reflector located at a second end of the fiber that reflects light propagated along the inner core from the first end towards the second end back to the first end. Additionally or alternatively the fiber comprises an optical reflector at the second end that reflects light propagated along the ring core from the first end to the second end back to the first end.

In some embodiments of the invention, the fiber comprises a deformation that causes a relatively large portion of optical energy introduced into the ring core at the first end to propagate along the ring core in propagation modes having a radial index substantially larger than that of the fundamental propagation mode of the ring core.

There is further provided a device for providing an intravascular ultrasound image of a vessel comprising an optic fiber according to an embodiment of the present invention and an optical system that introduces light at the first end of the fiber that propagates in the inner core and light that propagates in the outer core.

In some embodiments of the invention, the optical system illuminates a surface of the ring core at the first end with light at angles of incidence to the surface that are relatively large. Optionally, the angles of incidence are relatively close to an acceptance angle for the ring core corresponding to a numerical aperture of the core.

In some embodiments of the invention, the fiber comprises a deformation that causes a relatively large portion of optical energy introduced into the ring core at the first end to propagate along the ring core in propagation modes having a radial index substantially larger than that of the fundamental propagation mode of the ring core. Optionally, the deformation comprises a bend in the fiber. Optionally, the bend has a radius less than or equal to 3 cm. Optionally, the bend has a radius less than or equal to 2 cm. Optionally, the bend has a radius less than or equal to 1 cm.

In some embodiments of the invention, the relatively large portion of optical energy propagates in propagation modes having radial indices equal to or greater than three plus the radial index of the fundamental mode.

In some embodiments of the invention, the relatively large portion of optical energy propagates in propagation modes having radial indices equal to or greater than five plus the radial index of the fundamental mode.

In some embodiments of the invention, the relatively large portion of optical energy propagates in propagation modes having radial indices equal to or greater than six plus the radial index of the fundamental mode.

In some embodiments of the invention, at least 40% of the optical energy propagates in higher radial index modes.

In some embodiments of the invention, at least 60% of the optical energy propagates in the higher radial index modes.

In some embodiments of the invention, at least 80% of the optical energy propagates in the higher radial index modes.

### BRIEF DESCRIPTION OF FIGURES

Non-limiting examples of embodiments of the present invention are described below with reference to figures attached hereto, which are listed following this paragraph. In the figures, identical structures, elements or parts that appear in more than one figure are generally labeled with a same numeral in all the figures in which they appear. Dimensions of components and features shown in the figures are chosen for convenience and clarity of presentation and are not necessarily shown to scale.
Figs. 1A and 1B schematically show perspective and longitudinal cross section views respectively of an US imaging guidewire (IG) comprising a dual transmission fiber, in accordance with an embodiment of the present invention;
Fig. 2A schematically shows the IG shown in Figs 1A and 1B being used to image the lumen of a blood vessel, in accordance with an embodiment of the present invention;
Fig. 2B shows a bar graph of the energy distribution among propagation modes with which a light pulse of power light propagates in a dual transmission fiber when light in the light pulse is inserted into the fiber in accordance with an embodiment of the present invention;
Fig. 2C shows a graph of the propagation mode energy distribution for a power light pulse when light in the pulse is inserted into a dual transmission fiber using a "homogeneous insertion configuration", for which the inserted light is substantially parallel to the fiber axis and has a substantially uniform intensity over the area of the proximal end;
Fig. 2D shows a bar graph of the propagation mode energy distribution for a power light pulse when light in the pulse is inserted into a compound fiber using a "homogeneous insertion configuration", and the fiber has a bend, in accordance with an embodiment of the present invention;
Fig. 2E shows a graph of percent of energy absorption by an absorber on the surface of a ring core in a dual transmission fiber as a function of radius of a bend formed in the fiber, in accordance with an embodiment of the present invention;
Figs. 3A and 3B schematically show perspective and cross section views respectively of an IG comprising a dual transmission fiber having a plurality of acoustic transducers, in accordance with an embodiment of the invention; and
Fig. 4 schematically shows an IG having an acoustic transducer comprising a "sectored absorber", which is an annular shaped absorber divided into sectors, each of which may be excited to radiate ultrasound independently of the others, in accordance with an embodiment of the present invention.

### DETAILED DESCRIPTION OF EXEMPLARY EMBODIMENTS

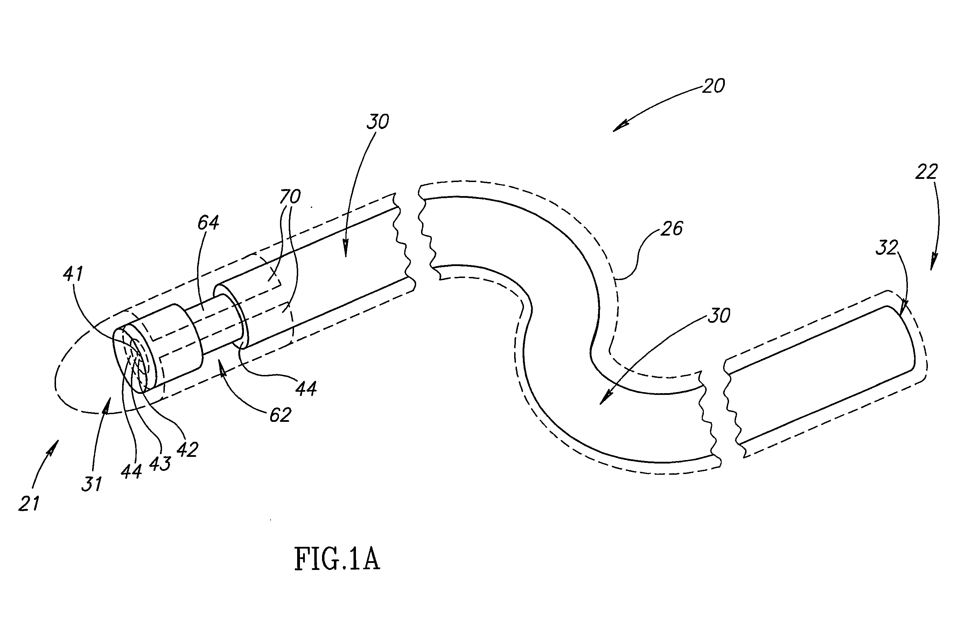

Fig. 1A schematically shows a perspective view of an IG 20 having a distal end 21 and a proximal end 22, in accordance with an embodiment of the invention. IG 20 optionally comprises an outer guide tube 26 shown in dashed lines and a dual transmission fiber 30 adapted for transmitting ultrasound into a lumen and for receiving echoes of the transmitted ultrasound.

To facilitate threading IG 20 through the vascular system and positioning its distal end 21 in a region of a vessel to be examined, guide tube 26 is optionally formed from metal or a suitable polymer such as polyimide using any of various methods and devices known in the art so that it functions as a guidewire. To this end, guide tube 26 is relatively flexible and bendable near distal end 21 so that IG 20 may be controlled to negotiate bends and turns in the vascular system. The guide tube gradually becomes more rigid and less bendable towards proximal end 22 to provide the IG with "pushability". Optionally, guide tube 26 is equipped at distal end 21 with a flexible a-traumatic tip (not shown) similar to those known in the art of guidewires. Such tips are conventionally formed in the shape of a spring and often comprise radio-opaque fiducials to facilitate locating the tip when it is introduced into a patient's body.

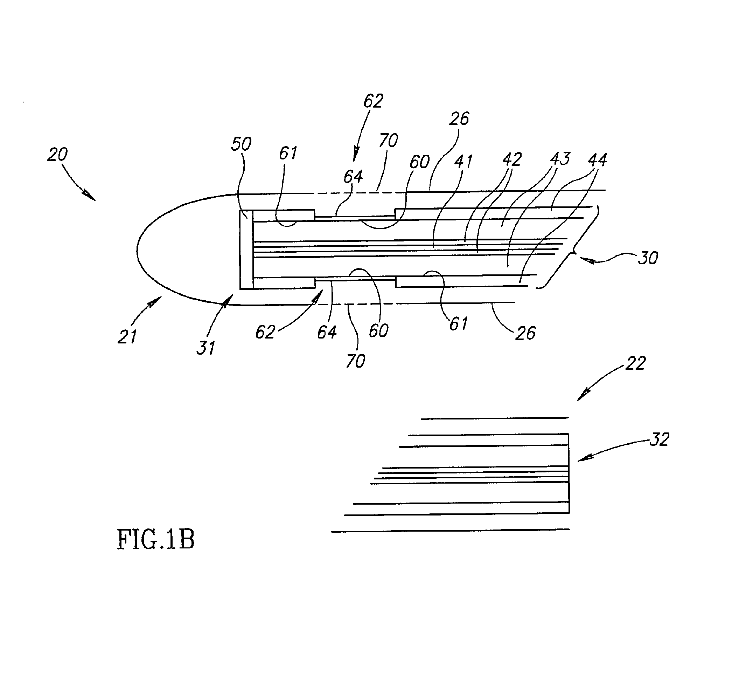

Dual transmission fiber 30 has distal and proximal ends 31 and 32 near distal and proximal ends 21 and 22 respectively of IG 20 and is schematically shown as seen through outer tube 26. Fiber 30 is, optionally, glued to guide tube 26 using any of various materials and methods known in the art to maintain the position of the fiber in the guide tube. In some embodiments of the invention, space between fiber 30 and the wall of guide tube 26 is filled with a suitable material, such as any of various gels known in the art, that supports ultrasound propagation. Fig. 1B schematically shows a longitudinal cross section view of IG 20 taken along the length of the IG.

As seen more clearly in Fig. 1B dual transmission fiber 30 comprises an inner, single mode (SM), core 41, an inner core cladding 42, a ring core 43 and an outer cladding 44. Inner core cladding 42 has an index of refraction less than that of inner core 41 and ring core 43. Outer cladding 44 optionally has an index of refraction less than that of ring core 43. An optical reflector 50, which reflects light, *i*.*e*. signal light, transmitted along inner core 41 from proximal end 32 of fiber 30, is formed or coupled to distal end 31 of the fiber. Optionally, reflector 50 also reflects light, *i.e.* power light transmitted along ring core 43 from the proximal end of the fiber.

In accordance with an embodiment of the invention, an optionally annular portion of outer cladding 44 is removed to expose a region 60 (Fig. 1B) of outer surface 61 of ring core 43. An acoustic transducer 62 is optically coupled to the exposed region of the ring core. Transducer 62 comprises an optical absorber 64 adhered to ring core 43 that absorbs optical energy from power light propagated along ring core 43 and converts the absorbed optical energy to ultrasound energy and transmits the ultrasound energy to the outside of the IG. Optionally, guide tube 26 is formed with at least one window 70 formed for example, from a material, such as polyimide, that is substantially transparent to ultrasound transmitted by optical absorber 64.

In some embodiments of the invention, optical absorber 64 comprises a metal. Optionally, the metal is chosen from the group of metals consisting of Aluminum, Copper, Titanium and Silver. In some embodiments of the invention, absorber 64 comprises a plurality of different layers, with an inner layer that serves to provide good bonding of the absorber to the ring core surface and an outer layer or layers optionally providing relatively good conversion of optical energy to ultrasound energy. Optionally, the absorber comprises two layers, an inner layer formed from Ti and an outer layer formed from A1. Ti has relatively good adherence to glass and A1 is a relatively good converter of optical energy to ultrasound energy. The inventors have found that a Ti-A1 absorber for which the Ti layer has a thickness substantially equal to about 0.3 micrometers and the A1 layer a thickness equal to about 0.7 micrometers is relatively convenient to manufacture, is mechanically relatively stable and provides relatively efficient conversion of optical energy to ultrasound energy.

Optionally, absorber 64 is formed from a mixture of a metal powder and a binder. In some embodiments of the invention, the powder is a silver nano-powder, such as marketed by Cima NanoTech Israel, LTD. of Caesarea, Israel. Optionally, an average diameter of the silver particles in the nano-powder is about 60 nanometers. In some embodiments of the invention, the binder is a UV curable material such as a conformal polymer coating of the chemical class Urethane (Meth) Acrylate marketed under the trade name Multi-Cure^{®} 9-984-B marketed by Dymax, of Torrington, CT, USA. A mixture of the silver powder and UV binder is applied to exposed region 60 (Fig. 1B) of ring core 43 and the UV binder cured by exposure to UV light to form absorber 64.

By way of a numerical example, in an embodiment of the invention, dual transmission fiber 30 is optionally a step index fiber for which inner core 41 has a diameter equal to about 9 micrometers, and a numerical aperture (NA) equal to about 0.132. Inner core cladding 42 has an outer diameter of about 40 micrometers. Ring core 43 optionally has an outer diameter of about 105 micrometers and a NA equal to about 0.25. Cladding 44 that covers ring core 43 is, optionally, at least about 10 micrometers thick. The fiber is, optionally, covered in a suitable protective outer buffer layer, resulting in an outer diameter for the fiber equal to about 146 micrometers. Guide tube 26 that sheaths dual transmission fiber 30 has an outer diameter of optionally equal to about 1 French, *i*.*e*. about 355 micrometers. In Figs. 1A and 1B and figures that follow, for convenience of presentation, cladding 44 and an outer protective, buffer, layer are not shown as distinct from each other. Absorber 64 optionally comprises a 0.3 micrometer inner layer of Ti and a 0.7 micrometer thick outer layer of A1 and covers a length of the outer surface 61 of ring core 43 equal to about 2 mm. Signal light optionally comprises green light at a wavelength equal to about 532 nm and power light optionally comprises IR light at a wavelength of about 1064 nm.

It is noted that an IG similar to IG 20 in accordance with an embodiment of the invention and the numerical example given above, has a diameter substantially smaller than that of a typical IVUS catheter. As a result, the IG may be used to probe blood vessels in the body having diameters substantially smaller than those that may be probed using a conventional IVUS catheter. For some applications, such as for examining small delicate blood vessels in the eye, an IG, in accordance with an embodiment of the invention, may be manufactured having a diameter less than that given in the numerical example.

A smaller IG in accordance with the invention may, for example, be provided by using a dual transmission fiber similar to optic fiber 30 having a cross section diameter equal to about 150 microns sheathed in a guide tube 26 having a diameter equal to or less than about 200 microns. Optionally, the guide tube is formed directly on the fiber from, for example polyimide. Optionally, the guide tube is formed directly on outer ring core 43 and replaces and functions as cladding for the outer core as well as guide tube for the IG.

It is further noted that an IG, in accordance with an embodiment of the invention may be used as an imaging guidewire for any of the purposes and with any other devices for which a conventional guidewire, such as for example a 1 French (0.014 inch, 355 micrometers) imaging guidewire, is used. For example, an IG, in accordance with an embodiment of the invention, may be used with a conventional balloon therapy catheter to guide insertion of the catheter into a cardiac blood vessel.

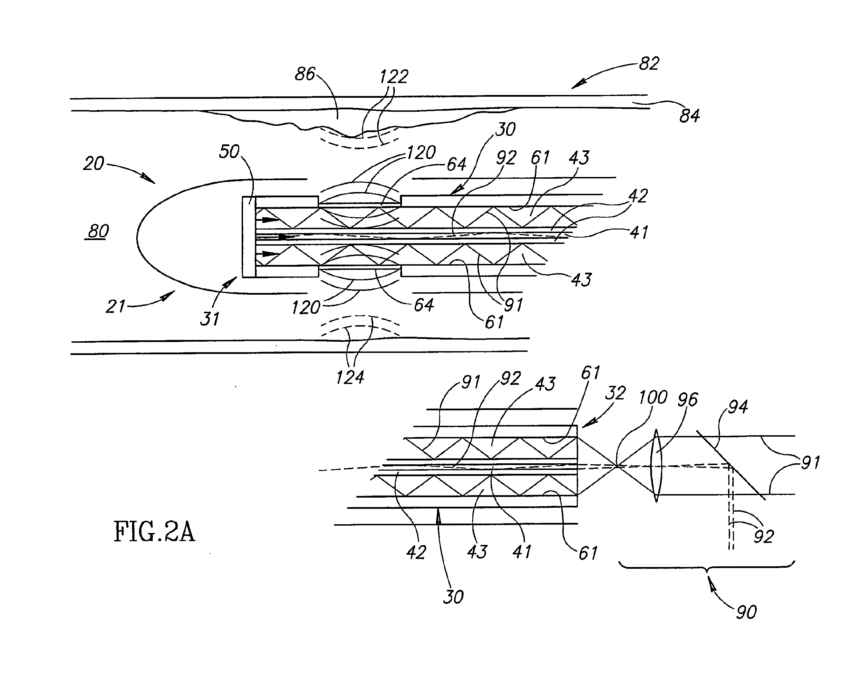

Fig. 2A schematically shows IG 20 being used, by way of example, to image a lumen 80 of a patient's blood vessel 82, in accordance with an embodiment of the invention. Blood vessel 82 has walls 84 and is compromised by plaque 86 which narrows the blood vessel lumen. To image lumen 80, IG 20 is threaded into and through the patient's vascular system so that distal end 21 of the IG is located inside lumen 80. Proximal end 22 of IG 20 remains, of course, outside the patient's body.

Once in position, an appropriate optical system 90 introduces power light for stimulating absorber 64 to radiate ultrasound into lumen 80 at a desired frequency and signal light for sensing acoustic energy in the radiated ultrasound that is reflected by walls 84 into fiber 30 in a neighborhood of the fiber's proximal end 32. Optical system 90 receives the power light, schematically represented by lines 91, and signal light, schematically represented by dashed lines 92 from suitable light sources (not shown), such as lasers and/or LEDs. Power light 91 optionally comprises pulsed light having a pulse repetition rate substantially equal to the desired frequency of the transmitted ultrasound. Optionally, signal light 92 comprises CW light.

Ultrasound radiated by absorber 64 responsive to pulses of power light 91 that reach the absorber is represented by solid curved lines 120. Some of radiated ultrasound 120 propagates towards inner core 41 and when it reaches the inner core it causes a local change in the index of refraction of the inner core, which in turn generates a phase change in signal light 92 propagating in the inner core. The phase change, propagates to proximal end 32, where it is detected using any of various methods and devices known in the art such as interferometric methods and devices.

Some of radiated ultrasound 120 propagates to walls 84 of blood vessel 82. Echoes of the radiated ultrasound reflected back towards fiber 30 by the walls are schematically represented by dashed curved lines 122 and 124. Echoes 122 are reflected by plaque deposit 86 and echoes 124 are reflected by healthy tissue. When echoes 122 and 124 reach inner core 41 they generate phase changes in signal light 92 that propagate to proximal end 32 where they are detected similarly to the phase change generated in signal light 92 by ultrasound 120. The signals are processed using methods known in the art, such as those described in PCT Patent Publication WO 03/057061 to provide, *inter alia,* a measure of the narrowing in the diameter of lumen 80 caused by plaque deposit 86.

In accordance with an embodiment of the invention, optical system 90 is designed to introduce signal light 92 and pulses of power light 91 into fiber 30 so that substantially all and substantially only signal light propagates along inner core 41. In addition, the optical system is designed so that substantially all power light 91 enters and is propagated along ring core 43 and that a relatively large portion of the power light propagates in higher order propagation modes. The inventors have found that for a pulse of power light 91 propagating in a relatively high order radial propagation mode, energy in the pulse spends a relatively large portion of the time close to outer surface 61 of power core 43. In addition, light in the pulse tends to be incident on outer surface 61 of ring core 43 at greater angles of incidence as the radial index of the mode increases. Both the relatively large concentration of optical energy near outer surface 61 of ring core 43 and increased angle of incidence of light on the outer surface of the ring core tends to increase absorption of optical energy from the pulse by absorber 64 when the pulse reaches the absorber. As a result, in accordance with an embodiment of the invention, optical energy transmitted along fiber 30 is relatively efficiently converted to radiated ultrasound energy.

In some embodiments of the invention, a relatively large portion of the power light propagates in propagation modes having radial indices equal to or greater than three plus the radial index of a fundamental mode of the ring core. Optionally, a relatively large portion of the power light propagates in propagation modes having radial indices equal to or greater than five plus the radial index of the fundamental mode. Optionally, a relatively large portion of the power light propagates in propagation modes having radial indices equal to or greater than five plus the radial index of the fundamental mode. In some embodiments of the invention, the relatively large portion of the power light comprises 40% of the optical energy in the power light. Optionally, the large portion comprises 60% of the optical energy in the power light. Optionally, the large portion comprises 80% of the optical energy in the power light.

In some embodiments of the invention, optical system 90 comprises a combiner, represented by a beam-splitting mirror 94, and focusing optics represented by a lens 96. Combiner 94 receives light from a source (not shown) of signal light 92 and a source (not shown) of power light 91 and directs the received light to lens 96. Lens 96 focuses the light to a focal spot 100 outside of fiber 30, which is displaced from proximal end 32 of the fiber along the axis of the fiber. Focal spot 100 is positioned relative to proximal end 32 of fiber 30 so that power light 91 that leaves the focal spot illuminates substantially all but substantially only inner core 41, cladding 42 and ring core 43 and signal light 92 illuminates substantially all but substantially only inner core 41. The half cone angles of power light 91 and signal light 92 focused to focal spot 100 are substantially equal to the numerical apertures of ring core 43 and inner core 41 respectively. As a result, substantially all signal light 92 that illuminates proximal end 32 of fiber 30 enters and propagates along inner core 41 to distal end 31 of the fiber. Because the cone of power light 91 that illuminates proximal end 32 of fiber 30 has a relatively large half cone angle, pulses of power light 41 have a tendency to propagate along fiber 30 in propagation modes having relatively high radial indices, and efficiency of coupling energy from pulses to absorber 64 is relatively high.

The insertion configuration described above by which optical system 90 inserts a pulse of power light 91 into fiber 30 is referred to as a "wide-angle insertion configuration". An insertion configuration for which light is incident at proximal end 32 of fiber 30 at angles of incidence substantially equal to 0° (*i*.*e*. parallel to the axis of the fiber) and that has substantially uniform intensity over the area of the proximal end, is referred to as a "homogenous insertion configuration". A light pulse introduced into fiber 30 using a homogenous insertion configuration tends to propagate along the fiber in relatively low order propagation modes.

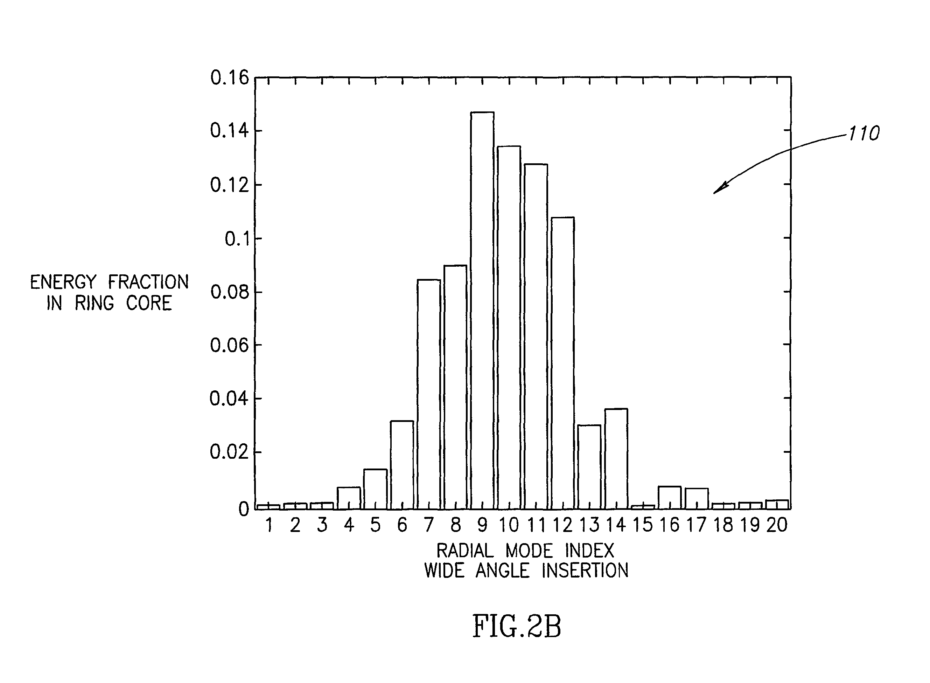

Fig. 2B shows a bar graph 110 of the energy distribution among propagation modes with which a light pulse of power light 91 propagates in fiber 30 when light in the light pulse is input to the fiber in a wide-angle insertion configuration by optical system 90 as described above. The abscissa of graph 110 gives the radial mode indices of the propagation modes and the ordinate, the fraction of the total energy in the light pulse in the modes. The energy distribution is calculated for fiber 30 having specifications defined in the above numerical example, for focal spot 100 located about 0.25 mm in front of proximal end 32 of the fiber and power light 91 focused to the focal spot with a maximum convergence angle equal to about the acceptance angle of the ring core of the fiber.

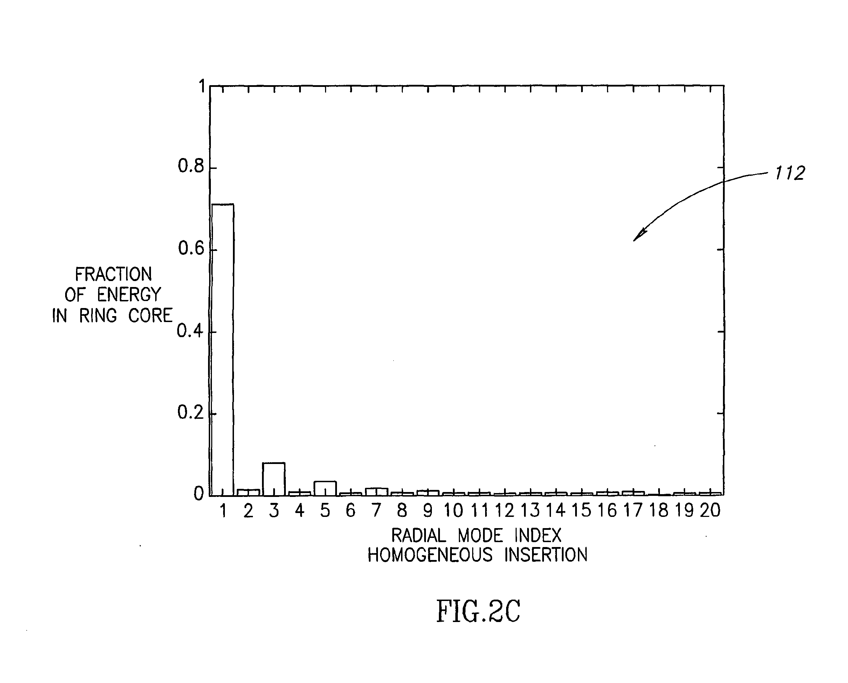

For comparison, Fig. 2C shows a bar graph 112 of the "propagation mode energy distribution" for a power light pulse inserted into fiber 30 using a homogeneous insertion configuration. Graph 112 shows that for a homogenous insertion configuration, most of the inserted energy propagates in the lowest radial index (index 1) propagation mode. On the other hand, graph 110 shows that for wide-angle insertion, most of the inserted optical energy propagates in propagation modes having radial indices in a range from about 6 to about 13. Substantially none of the energy propagates in the lowest radial index modes.

For a wide angle insertion configuration, the inventors have found that for a fiber, in accordance with an embodiment of the invention, having the specifications given in the above numerical example, from about 40% to about 50% of the optical energy in a pulse of power light 91 is absorbed by absorber 64 if the pulse passes by the absorber once, is not reflected by reflector 50 and is allowed to exit the fiber from its distal end 31. Absorption is substantially larger if the IR power light 91 pulses are reflected back from distal end 31 by reflector 50 so that each pulse of IR power light 91 passes by absorber 64 twice. For a power light pulse inserted into fiber 30 using a homogenous insertion configuration, "single pass absorption" is estimated to be between about 10% to about 15%.

In some embodiments of the invention, lens 96 comprises a holographic lens that is configured to illuminate proximal end 32 of fiber 30 with power light in an insertion configuration that excites a particular desired high order propagation mode for the power light. For example, the holographic lens may be designed so that power light 91 illuminates proximal end 32 of fiber 30 substantially only in a region near the outer diameter of ring core 43 and at angles of incidence close to the angle of acceptance corresponding to the numerical aperture of the ring core. Optionally, the power light pattern generated by the holographic lens has a rotational symmetry greater than one.

In some embodiments of the invention, light system 90 is configured to introduce a power light pulse into fiber 30 using a homogenous insertion configuration. To stimulate propagation of the light pulse at relatively high order propagation modes, in accordance with an embodiment of the invention, fiber 30 is mechanically deformed. The inventors have found that by suitably deforming fiber 30 near its proximal end 32, some of the optical energy in light that would otherwise propagate along the fiber in a relatively low order mode is coupled into relatively high order propagation modes. Optionally, deforming the fiber comprises bending the fiber in a region close to proximal end 32 of the fiber.

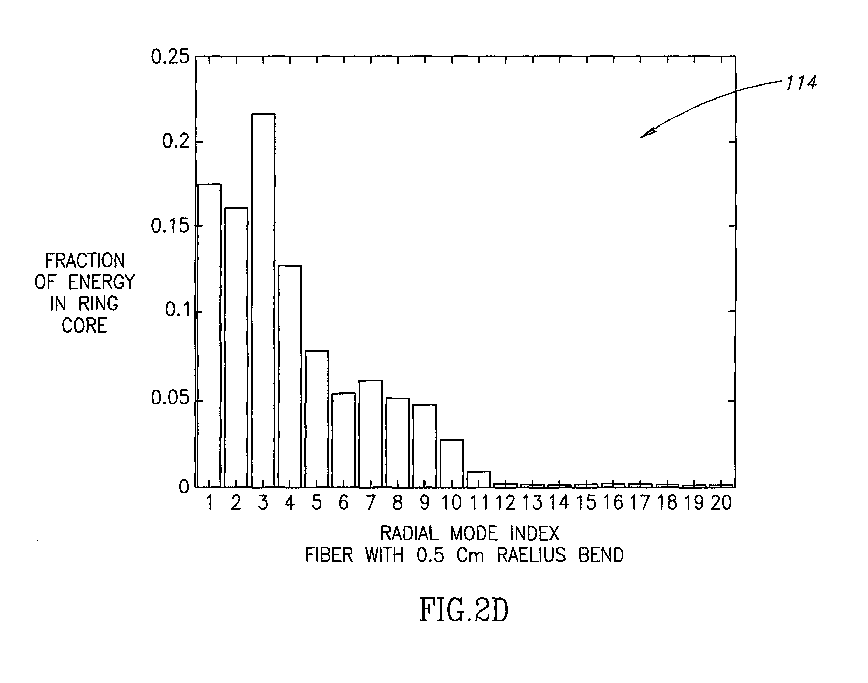

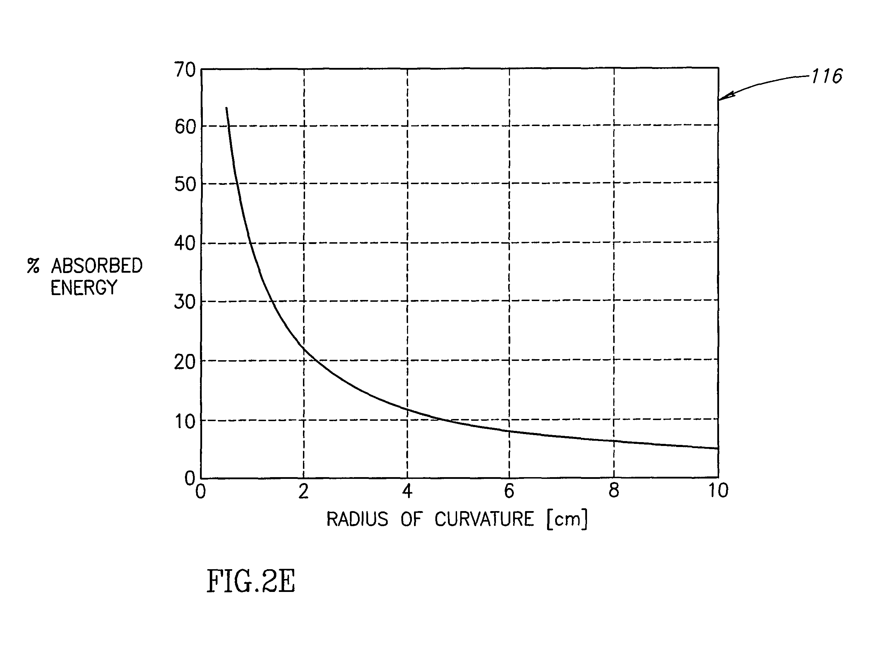

Fig. 2D shows a bar graph 114 of the propagation mode energy distribution for a pulse of power light 91 inserted into fiber 30 using a homogeneous insertion configuration when the fiber has a bend of radius 0.5 cm optionally near its proximal end 32. Comparison of bar graph 110 with bar graph 112 indicates that the bend is effective in redistributing about 75% of the energy in the first radial mode into higher radial modes. Fig. 2E shows a graph 116 of percent of energy absorption calculated for absorber 64 from a pulse of power light 91 introduced into fiber 30 using a homogenous insertion configuration as a function of radius of a bend in the fiber formed near its proximal end 32. The calculation assumes that light in a pulse of power light 91 is absorbed with 100% efficiency if the light is incident on the absorber and that the absorber has specifications defined in the above numerical example. From graph 116 it is seen that as the bend radius decreases, and severity of fiber deformation increases, more energy in the pulse is distributed to higher propagation modes and the percent of energy absorption increases.

It is noted that whereas in the above exemplary embodiment, inner core 41 and ring core 43 are separated by an inner core cladding, cladding 42, in some embodiments of the invention, an IG comprises a dual transmission fiber having an inner core and ring core that are not separated by a cladding. For such embodiments of the invention, the ring core functions not only as a core for the transmission of power light but also as a cladding for the inner core. Coupling of power light propagated along the ring core to an ultrasound absorber formed on the outer surface of the ring core is subject to considerations similar to those discussed above for dual transmission fiber 30 shown in Figs. 1A-2A. Efficiency of coupling optical energy from a pulse of power light propagating along the ring core to the absorber tends to improve as the orders of the propagation modes of the pulse increase. Propagation of energy in the pulse can be distributed to higher propagation modes, in accordance with an embodiment of the invention, by appropriately configuring an insertion configuration for the light pulse and/or deforming the cable.

It is expected however, that in general, optical energy in a power pulse will be more efficiently coupled to an absorber on the outer surface of a ring core for a dual transmission fiber having an inner core cladding between its inner and ring cores than for a dual transmission fiber absent an inner core cladding.

As noted above, coupling of optical energy from a power light pulse to the absorber on an outer surface of a ring core is a function of concentration of optical energy in the pulse close to the outer surface and angle of incidence of light in the pulse on the outer surface. However, coupling of optical energy in the light pulse to the absorber is also a function of a number of times light in the light pulse is incident on the absorber. For a dual transmission fiber having an inner core cladding, power light propagating along the fiber's ring core bounces back and forth between the outer surface of the ring core and the interface between the ring core and the inner cladding (which is along an inner surface of the ring core). For a dual transmission fiber absent an inner cladding on the other hand, power light propagating along the ring core bounces back and forth only between different regions of the outer surface. An average path length between bounces is less for the dual transmission fiber having an inner core cladding than for a dual transmission fiber absent an inner core cladding. As a result, per unit length of the outer surface of the ring core, light in a power light pulse is incident more frequently on the outer surface for the dual transmission fiber having an inner core cladding than for a dual transmission fiber absent an inner core cladding. Therefore, for a given length of absorber on the ring core outer surface, power light is incident more frequently on the absorber and energy coupling from the power light to the absorber is more efficient for the dual transmission fiber having an inner core cladding than for a dual transmission fiber absent an inner core cladding. In addition, a dual transmission fiber absent an inner core cladding in general supports a smaller number of the higher order propagation modes that are relatively efficient in coupling energy to an absorber than a dual transmission fiber having an inner core cladding and substantially a same outer diameter as the dual transmission fiber absent the inner core cladding.

In some embodiments of the invention, an IG comprises a plurality of acoustic transducers along its length so that when the IG is positioned inside the lumen of a blood vessel it can be used to image the lumen at a plurality of different locations along the length of the lumen without having to move the IG.

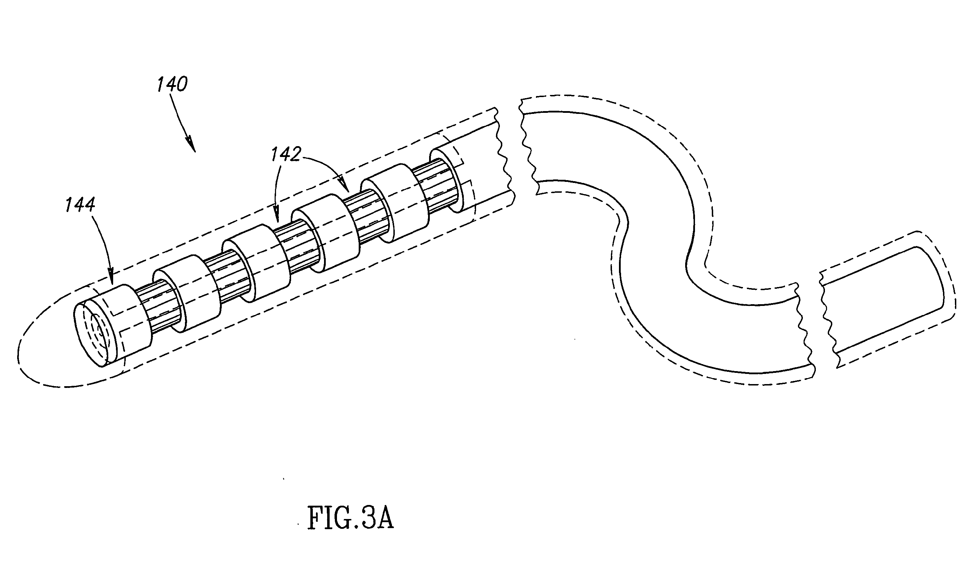

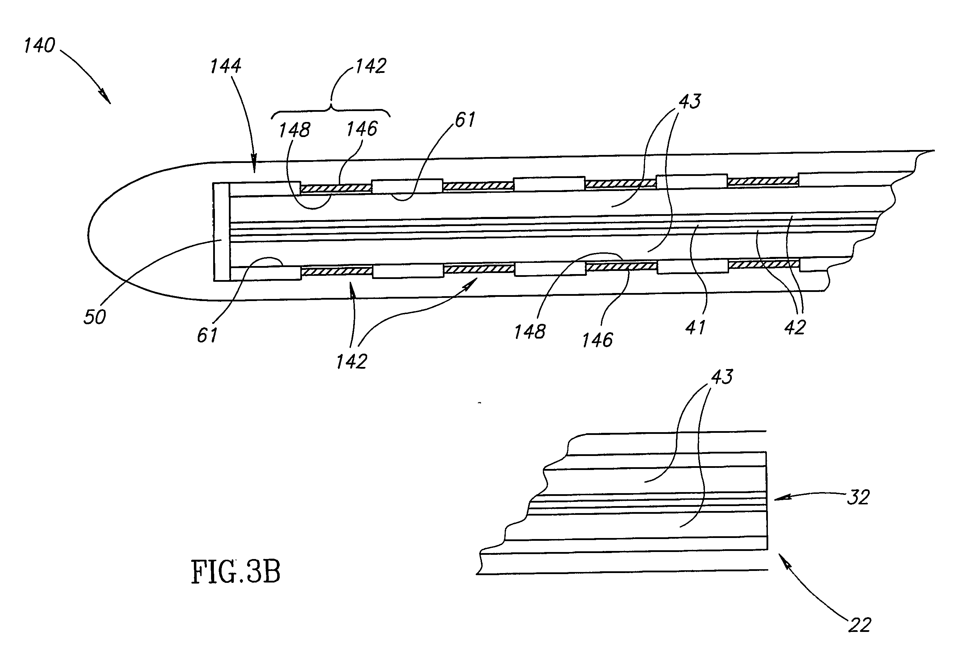

Figs. 3A and 3B schematically show a perspective and cross section view respectively of an IG 140 comprising a plurality of acoustic transducers 142 coupled to a dual transmission fiber 144, in accordance with an embodiment of the present invention. Except for the plurality of acoustic transducers, IG 140 is similar to IG 20. Each acoustic transducer 142 comprises an absorber 146 and, optionally, an optical band-pass filter 148 (Fig. 3B) sandwiched between the absorber and the outer surface 61 of ring core 43 that transmits light in a different band of wavelengths. The bandwidths of filters 148 associated with different acoustic transducers 142 are, optionally, substantially non-overlapping. A given acoustic transducer 142 is controllable to radiate ultrasound independently of the other transducers 142 by transmitting power light along ring core 43 that is transmitted substantially only by optical filter 148 associated with the given transducer.

In the exemplary embodiments of the invention described above, the acoustic transducers comprise an absorber that has a shape of an unbroken, complete, annulus. In some embodiments of the invention, an IG comprises a "sectored acoustic transducer" coupled to a ring core of a dual transmission fiber. The sectored acoustic transducer comprises an array absorbing regions, hereinafter "absorbing sectors", configured in a substantially annular array around the circumference of the ring core.

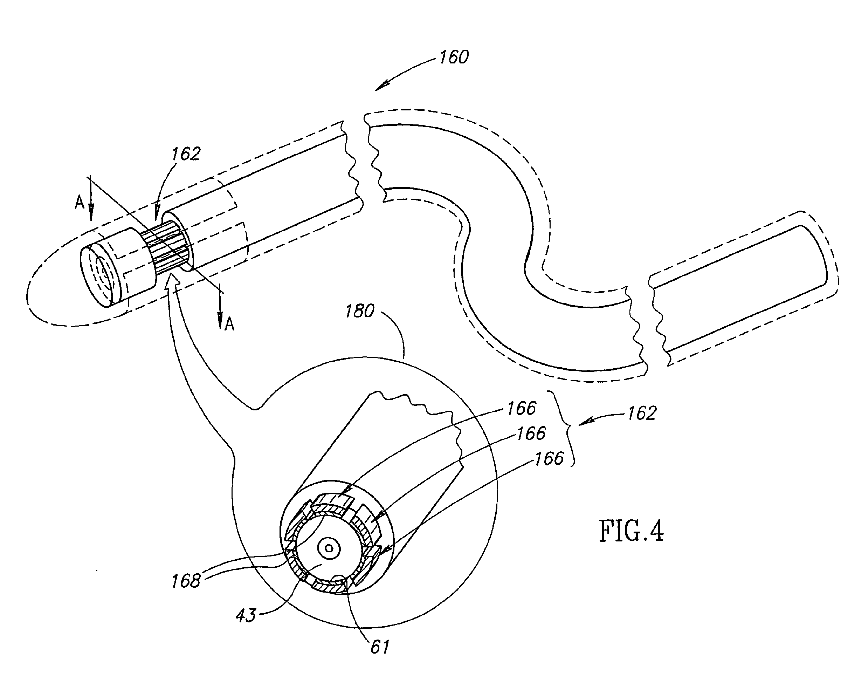

Fig. 4 schematically shows an IG 160 comprising a sectored acoustic transducer 162 comprising a plurality of absorbing sectors 166, in accordance with an embodiment of the invention. An inset 180 schematically shows an enlarged image of acoustic transducer 162. An optical band pass filter 168 is sandwiched between each absorbing sector 166 and outer surface 61 of ring core 43. Each band pass filter 168 optionally transmits light in a different substantially non-overlapping band of wavelengths. A given absorbing sector 166 is controllable to radiate ultrasound independently of the other sectors 166 by transmitting power light along ring core 43 that is transmitted substantially only by optical filter 168 associated with the given absorbing sector. By sequentially exciting different absorbers 166 to radiate ultrasound, features of the walls of a blood vessel lumen can be located as a function of azimuth angle relative to the axis of IG 160. For example, a lesion in the blood vessel wall can be determined to be located in a particular azimuthal region of the blood vessel wall.

In some embodiments of the invention, an IG comprises a plurality of absorbers, and has by way of example a configuration similar to that of IG 140 or 160, except that each of the absorbers is not coupled to an optical band-pass filter having a different band-pass. When power light is transmitted along a ring core of the IG to excite acoustic vibrations in the absorbers, all the absorbers are simultaneously excited to transmit ultrasound. However, in accordance with an embodiment of the invention, each of the plurality of absorbers is formed so that it has a resonant frequency of vibration different from that of the other absorbers. For example, each of the absorbers may be formed having a dimension different from that of the other absorbers, which determines a resonant frequency for the absorber different from the resonant frequencies of the other absorbers. As a result, when excited by a pulse of power light each of the absorbers transmits ultrasound at a different frequency. Reflections of the ultrasound transmitted by each of the different absorbers from features of a lumen of a blood vessel in which the IG is located modulate signal light at a different frequency. The modulations of the signal light at each of the different frequencies are separately detected using any of various methods and apparatus known in the art and are used to generate images of different regions of the lumen.

It is noted that whereas an IG in accordance with the present invention has been described as being used for probing the lumen of a blood vessel, an IG in accordance with the invention can of course be used for probing the lumens of other conduits in the body, such as a bile duct or the urethra. An IG in accordance with an embodiment of the invention may of course also be used to probe other than biological conduits.

In the description and claims of the present application, each of the verbs, "comprise" "include" and "have", and conjugates thereof, are used to indicate that the object or objects of the verb are not necessarily a complete listing of members, components, elements or parts of the subject or subjects of the verb.

The present invention has been described using detailed descriptions of embodiments thereof that are provided by way of example and are not intended to limit the scope of the invention. The described embodiments comprise different features, not all of which are required in all embodiments of the invention. Some embodiments of the present invention utilize only some of the features or possible combinations of the features. Variations of embodiments of the present invention that are described and embodiments of the present invention comprising different combinations of features noted in the described embodiments will occur to persons of the art. The scope of the invention is limited only by the following claims.

## Claims

1. An optic fiber adapted for ultrasound imaging of the lumen of a vessel comprising:
an inner core (41) for transmitting light having an index of refraction that changes responsive to acoustic energy incident thereon;
a ring core (43) concentric with the inner core for transmitting light;
a cladding material (42) between the inner and ring cores that has an index of refraction smaller than the index of refraction of the material from which the inner core is formed; and
at least one acoustic transducer (62) comprising absorbing material (64) formed on the surface of the ring core that absorbs optical energy transmitted along the ring core and generates ultrasound responsive thereto.

2. An optic fiber according to claim 1 wherein the cladding material has an index of refraction smaller than the index of refraction of the material from which the ring core is formed.

3. An optic fiber according to claim 1 or claim 2 wherein the inner core is a single mode core.

4. An optic fiber according to any of the preceding claims wherein the absorber comprises a metal.

5. An optic fiber according to claim 4 wherein the metal is chosen from the group of metals consisting of: aluminum, copper, silver, gold and titanium.

6. An optic fiber according to claim 4 or claim 5 wherein the absorber comprises a metallic powder dispersed in a binding medium.

7. An optic fiber according to any of the preceding claims wherein a transducer of the at least one acoustic transducer comprises a transducer shaped in the form of an annulus concentric with the ring core.

8. An optic fiber according to any of the preceding claims wherein the at least one acoustic transducer comprises a plurality of acoustic transducers.

9. An optic fiber according to claim 8 wherein at least two of the acoustic transducers comprise an optical filter (148) located between the absorbing material and the inner core that transmits light in a wavelength band of light that is absorbed by the absorber and blocks light at wavelengths outside the band of wavelength.

10. An optic fiber according to claim 9 wherein each of the optical filters of at least two of the plurality of acoustic transducers transmit light in different bands of wavelengths.

11. An optic fiber according to claim 10 wherein each of the at least two different bands of wavelengths are substantially non-overlapping.

12. An optic fiber according to any of claims 8-11 wherein the plurality of acoustic transducers are configured in an annular array concentric with the ring core.

13. An optic fiber according to claim 12 wherein all the acoustic transducers of the plurality of transducers are substantially identical.

14. An optic fiber according to any of claims 8-13 wherein at least two of the absorbers when excited by a same at least one pulse of light generates ultrasound at different frequencies.

15. An optic fiber according to any of the preceding claims and comprising an external sheath (26) concentric with the ring core that provides the fiber with mechanical properties that enables the fiber to be inserted and navigated through a system of connected vessels comprising the vessel to position the at least one acoustic transducer in the lumen.

16. An optic fiber according to any of the preceding claims and comprising a first end (32) at which light is inserted into the inner and ring cores.

17. An optic fiber according to claim 16 and comprising an optical reflector (50) located at a second end (31) of the fiber that reflects light propagated along the inner core from the first end towards the second end back to the first end.

18. An optic fiber according to claim 16 or claim 17 and comprising an optical reflector at the second end that reflects light propagated along the ring core from the first end to the second end back to the first end.

19. An optic fiber according to any of the preceding claims wherein the fiber comprises a deformation that causes a relatively large portion of optical energy introduced into the ring core at the first end to propagate along the ring core in propagation modes having a radial index substantially larger than that of the fundamental propagation mode of the ring core.

20. A device for providing an intravascular ultrasound image of a vessel comprising:
an optic fiber according to any of claims 16-19; and
an optical system (90) that introduces light at the first end of the fiber that propagates in the inner core and light that propagates in the outer core.

21. A device according to claim 20 wherein the optical system introduces light into the ring core at angles of incidence close to a numerical aperture of the ring.

## Patentansprüche

1. Lichtleitfaser, geeignet für die Ultraschall-Bilderzeugung des Lumens eines Gefäßes, umfassend:
einen inneren Kern (41) zum Übertragen von Licht mit einem Brechungsindex, der sich als Reaktion auf die darauf auftreffende akustische Energie ändert;
einen zum inneren Kern konzentrischen Ringkern (43) zum Übertragen von Licht;
ein Hüllmaterial (42) zwischen den inneren Kern und dem Ringkern, das einen Brechungsindex aufweist, der geringer ist als der Brechungsindex des Materials, aus dem der innere Kern gebildet ist; und
mindestens einen Schallwandler (62), der einen Dämmstoff (64) umfasst, der auf der Oberfläche des Ringkerns gebildet ist und die optische Energie absorbiert, die an dem Ringkern entlang übertragen wird, und als Reaktion darauf Ultraschall erzeugt.

2. Lichtleitfaser nach Anspruch 1, wobei das Hüllmaterial einen Brechungsindex aufweist, der geringer ist als der Brechungsindex des Materials, aus dem der Ringkern gebildet ist.

3. Lichtleitfaser nach Anspruch 1 oder 2, wobei der innere Kern ein Monomode-Kern ist.

4. Lichtleitfaser nach einem der vorhergehenden Ansprüche, wobei der Absorber ein Metall umfasst.

5. Lichtleitfaser nach Anspruch 4, wobei das Metall aus der Gruppe von Metallen gewählt wird, die aus Aluminium, Kupfer, Silber, Gold und Titan besteht.

6. Lichtleitfaser nach Anspruch 4 oder 5, wobei der Absorber einen Metallstaub umfasst, der in einem Bindemedium dispergiert ist.

7. Lichtleitfaser nach einem der vorhergehenden Ansprüche, wobei ein Wandler des mindestens einen Schallwandlers einen Wandler umfasst, der in der Form eines Ringes ausgestaltet ist, der zu dem Ringkern konzentrisch ist.

8. Lichtleitfaser nach einem der vorhergehenden Ansprüche, wobei der mindestens eine Schallwandler eine Vielzahl von Schallwandlern umfasst.

9. Lichtleitfaser nach Anspruch 8, wobei mindestens zwei der Schallwandler ein optisches Filter (148) umfassen, das zwischen dem Dämmstoff und dem inneren Kern liegt und das Licht in einem Lichtwellenlängenband, das von dem Dämmstoff absorbiert wird, überträgt und Licht auf Wellenlängen außerhalb des Wellenlängenbandes blockiert.

10. Lichtleitfaser nach Anspruch 9, wobei jedes der optischen Filter von mindestens zweien der Vielzahl von Schallwandlern Licht in verschiedenen Wellenlängenbändern überträgt.

11. Lichtleitfaser nach Anspruch 10, wobei jedes der mindestens zwei verschiedenen Wellenlängenbänder im Wesentlichen nicht überlappend ist.

12. Lichtleitfaser nach einem der Ansprüche 8 bis 11, wobei die Vielzahl von Schallwandlern in einer ringförmigen Anordnung konfiguriert ist, die zum Ringkern konzentrisch ist.

13. Lichtleitfaser nach Anspruch 12, wobei alle Schallwandler der Vielzahl von Wandlern im Wesentlichen identisch sind.

14. Lichtleitfaser nach einem der Ansprüche 8 à 13, wobei mindestens zwei der Absorber, wenn sie von mindestens einem gleichen Lichtimpuls erregt werden, Ultraschall auf verschiedenen Frequenzen erzeugen.

15. Lichtleitfaser nach einem der vorhergehenden Ansprüche und umfassend eine äußerte Hülle (26), die zu dem Ringkern konzentrisch ist und der Faser mechanische Eigenschaften verleiht, die das Einführen und Leiten der Faser durch ein System aus verbundenen Gefäßen, zu denen das Gefäß zum Anordnen des mindestens einen Schallwandlers in dem Lumen gehört, zu ermöglichen.

16. Lichtleitfaser nach einem der vorhergehenden Ansprüche und umfassend ein erstes Ende (32), an dem Licht in den inneren Kern und den Ringkern eingeführt wird.

17. Lichtleitfaser nach Anspruch 16 und umfassend einen optischen Rückspiegel (50), der sich an einem zweiten Ende (31) der Faser befindet und Licht spiegelt, das sich an dem inneren Kern entlang vom ersten Ende zum zweiten Ende und zurück zum ersten Ende ausbreitet.

18. Lichtleitfaser nach Anspruch 16 oder 17 und umfassend einen optischen Rückspiegel am zweiten Ende, der Licht spiegelt, das sich an dem Ringkern entlang vom ersten Ende zum zweiten Ende und zurück zum ersten Ende ausbreitet.

19. Lichtleitfaser nach einem der vorhergehenden Ansprüche, wobei die Faser eine Verformung umfasst, die einen relativ großen Teil der optischen Energie, die am ersten Ende in den Ringkern eingeführt wird, dazu veranlasst, sich an dem Ringkern entlang in Ausbreitungsmodi auszubreiten, die einen Radialindex aufweisen, der wesentlich größer ist als derjenige des Grundausbreitungsmodus des Ringkerns.

20. Vorrichtung zum Bereitstellen eines intravaskulären Ultraschallbildes eines Gefäßes, umfassend:
eine Lichtleitfaser nach einem der Ansprüche 16 ä 19; und
eine Optik (90), die am ersten Ende der Faser Licht, das sich in dem inneren Kern ausbreitet, und Licht, das sich im äußerten Kern ausbreitet, einführt.

21. Vorrichtung nach Anspruch 20, wobei die Optik Licht in den Ringkern unter Einfallswinkeln nahe an der numerischen Apertur des Ringes einführt.

## Revendications

1. Fibre optique, adaptée pour l'imagerie par ultrason de la lumière d'un vaisseau, comprenant :
un noyau intérieur (41) pour transmettre de la lumière ayant un indice de réfraction qui change en réponse à l'énergie acoustique incidente sur celui-ci;
un noyau annulaire (43) concentrique par rapport au noyau intérieur pour transmettre la lumière ;
un matériau de gainage (42) entre les noyaux intérieur et annulaire qui présente un indice de réfraction inférieur à l'indice de réfraction du matériau dont est formé le noyau intérieur; et
au moins un transducteur acoustique (62), comprenant un matériau absorbant (64) formé sur la surface du noyau annulaire, qui absorbe l'énergie optique transmise le long du noyau annulaire et génère des ultrasons en réponse à celle-ci.

2. Fibre optique selon la revendication 1, dans laquelle le matériau de gainage présente un indice de réfraction inférieur à l'indice de réfraction du matériau à partir duquel est formé le noyau annulaire.

3. Fibre optique selon la revendication 1 ou 2, dans laquelle le noyau intérieur est un noyau monomode.

4. Fibre optique selon l'une quelconque des revendications précédentes, dans laquelle l'absorbeur comprend un métal.

5. Fibre optique selon la revendication 4, dans laquelle le métal est choisi dans le groupe de métaux composé : de l'aluminium, du cuivre, de l'argent, de l'or et du titane.

6. Fibre optique selon la revendication 4 ou 5, dans laquelle l'absorber comprend une poudre métallique dispersée dans un milieu de liaison.

7. Fibre optique selon l'une quelconque des revendications précédentes, dans laquelle un transducteur dudit au moins un transducteur acoustique comprend un transducteur façonné selon la forme d'un anneau concentrique par rapport au noyau annulaire.

8. Fibre optique selon l'une quelconque des revendications précédentes, dans laquelle ledit au moins un transducteurs acoustique comprend une pluralité de transducteurs acoustiques.

9. Fibre optique selon la revendication 8, dans laquelle au moins deux des transducteurs acoustiques comprennent un filtre optique (148) situé entre le matériau absorbant et le noyau intérieur qui transmet la lumière dans une bande de longueurs d'onde de lumière qui est absorbée par l'absorbeur et bloque la lumière à des longueurs d'onde en dehors de la bande de longueurs d'onde.

10. Fibre optique selon la revendication 9, dans laquelle chacune des fibres optiques d'au moins deux de la pluralité de transducteurs acoustiques transmet de la lumière dans différentes bandes de longueurs d'onde.

11. Fibre optique selon la revendication 10, dans laquelle chacune desdites au moins deux bandes différentes de longueurs d'onde est substantiellement sans recouvrement.

12. Fibre optique selon l'une quelconque des revendications 8 à 11, dans laquelle la pluralité de transducteurs acoustiques est configurée selon un arrangement annulaire concentrique par rapport au noyau annulaire.

13. Fibre optique selon la revendication 12, dans laquelle tous les transducteurs acoustiques de la pluralité de transducteurs sont substantiellement identiques.

14. Fibre optique selon l'une quelconque des revendications 8 à 13, dans laquelle au moins deux des absorbeurs, lorsqu'ils sont excités par au moins une même impulsion de lumière, génèrent des ultrasons à des fréquences différentes.

15. Fibre optique selon l'une quelconque des revendications précédentes, et comprenant une enveloppe externe (26) concentrique par rapport au noyau annulaire qui confère à la fibre des propriétés mécaniques qui permettent l'insertion et la navigation de la fibre à travers un système de vaisseaux connectés comprenant le vaisseau pour positionner ledit au moins un transducteur acoustique dans la lumière.

16. Fibre optique selon l'une quelconque des revendications précédentes, et comprenant une première extrémité (32) à laquelle la lumière est insérée dans les noyaux intérieur et annulaire.

17. Fibre optique selon la revendication 16, et comprenant un réflecteur optique (50) situé au niveau d'une deuxième extrémité (31) de la fibre qui réfléchit la lumière propagée le long du noyau intérieur de la première extrémité vers la deuxième extrémité et de retour à la première extrémité.

18. Fibre optique selon la revendication 16 ou 17, et comprenant un réflecteur optique à la deuxième extrémité qui réfléchit la lumière propagée le long du noyau annulaire de la première extrémité à la deuxième extrémité et de retour à la première extrémité.

19. Fibre optique selon l'une quelconque des revendications précédentes, dans laquelle la fibre comprend une déformation qui amène une part relativement grande d'énergie optique introduite dans le noyau annulaire à la première extrémité à se propager le long du noyau annulaire dans des modes de propagation ayant un indice radial substantiellement supérieur à celui du mode de propagation fondamental du noyau annulaire.

20. Dispositif pour fournir une image intravasculaire par ultrasons d'un vaisseau, comprenant :
une fibre optique selon l'une quelconque des revendications 16 à 19 ; et
un système optique (90) qui introduit de la lumière à la première extrémité de la fibre qui se propage dans le noyau intérieur et de la lumière qui se propage dans le noyau extérieur.

21. Dispositif selon la revendication 20, dans lequel le système optique introduit la lumière dans le noyau annulaire sous des angles d'incidence proches d'une ouverture numérique de l'anneau.
